Europäisches Patentamt

European Patent Office

Office européen des brevets

(11) Publication number: **0 170 696**
**B1**

# (12) EUROPEAN PATENT SPECIFICATION

(45) Date of publication of patent specification: **14.11.90**

(51) Int. Cl.⁵: **A 61 N 1/04**

(21) Application number: **85901183.5**

(22) Date of filing: **01.02.85**

(86) International application number:
**PCT/US85/00177**

(87) International publication number:
**WO 85/03448 15.08.85 Gazette 85/18**

(54) FIXATION MEANS FOR AN ENDOCARDIAL ELECTRODE.

(30) Priority: **03.02.84 US 576640**

(43) Date of publication of application:
**12.02.86 Bulletin 86/07**

(45) Publication of the grant of the patent:
**14.11.90 Bulletin 90/46**

(84) Designated Contracting States:
**DE FR GB NL SE**

(56) References cited:
**EP-A- 85 967**
**US-A-4 057 067**
**US-A-4 258 724**
**US-A-4 262 982**
**US-A-4 419 819**
**US-A-4 467 817**

**No relevant documents have been disclosed.**

(73) Proprietor: **Siemens Elema AB**
**Röntgenvägen 2**
**S-171 95 Solna 1 (SE)**
(84) **SE**

(73) Proprietor: **Siemens Aktiengesellschaft**
**Wittelsbacherplatz 2**
**D-8000 München 2 (DE)**
(84) **DE FR GB NL**

(72) Inventor: **McARTHUR, William, A.**
**27544 Lovage Court**
**Saugus, CA 91350 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Technical field

The invention relates to implantable endocardial leads and more particularly to endocardial leads having structures near their tips for effecting fixation in heart trabeculae.

### Background art

Endocardial leads having fixation structures near their electrode tips are old in the art. Specifically, an endocardial lead having pliant tines at the electrode tip is disclosed in U.S. Patent 3,902,501 to Citron and Dickhudt. Further, leads having triangularly-shaped wedges near the electrode tip have also been utilized to enhance fixation. These leads are usually venously inserted into the heart and their electrode tips located adjacent to the heart wall. The fixation structures such as tines or triangularly-shaped wedges become enmeshed in the heart trabeculae, thus tending to fix the electrode tip in a position so that it will remain in electrical contact with and adjacent to the heart wall. However, it is sometimes required to reposition the electrode tip within the heart, or remove the lead entirely from the heart. This is usually effected by applying tension to the lead so as to disengage the fixation device from the trabeculae and to pull the electrode tip away from the heart wall.

One problem that arises is that the tines of currently utilized tined leads, and the corner formed by the backward edge and lead encasing material of finned electrodes, tend to get caught in or hang up on chordae tendonae present in the heart. In other words, tines provide good fixation with heart trabeculae but snag on the chordae tendonae during repositioning. EP—A—0085967 discloses a lead with fins where repositioning is facilitated in that permanent fixation is achieved by tissue ingrowth into the lead tip and the fins only provide for temporary fixation in that they are biologically degradable. US—A—4 419 819 discloses another repositionable lead. The lead is provided with a length of tubing slipped over the lead body adjacent to its distal end and provided with slits along the lead axis direction. The tubing is compressed so that the slit portions of the tubing expand into tissue fixation lobes. The lobes regain tube form when a stylet wire is inserted into the lead and reexpand when the stylet wire is removed. The present invention solves this problem by providing a fixation device that will entangle with the trabeculae in as efficient a manner as current fixation devices while at the same time not snagging or hanging up on chordae tendonae during electrode tip withdrawal or repositioning.

### Disclosure of invention

The invention solves the above problem by providing an endocardial lead as defined in the independent claim.

In specific embodiments of the invention, the deflection means defines an edge which is in the form of an arc extending from the fin back edge to the encasing material. This arc can be chosen to have a radius substantially the same as the radius of a trabecula in an average patient but much larger than that of the chordae tendonae. This configuration provides positive fixation or engagement with the trabeculae while at the same time allowing the chordae tendonae to slide off and not catch in the fin during lead removal. The edge of the deflecting means, however, can assume other shapes such as those of a straight line, or a stepped edge.

In further embodiments of the invention, flexure resistant portions are provided in the fin itself in order to alter the flexure characteristics of the fin as pressure is provided from various directions.

### Breif description of drawings

Figure 1 is a side view of an endocardial electrode tip according to the invention;

Figure 1A is an enlarged view of one fin shown in Figure 1 showing the relationship of the various parts of the fin to each other and the encasing material;

Figure 2 shows a further embodiment of the invention;

Figure 3 shows a cross-sectional view of the fin taken along line 3-3 of Figure 2;

Figure 4 shows a still further embodiment of the invention; and

Figure 5 is a cross-sectional view taken along line 5-5 of Figure 4.

### Best mode for carrying out the invention

Detailed illustrative embodiments of the invention disclosed herein exemplify the invention and are considered to be the best embodiments for such purposes. They are provided by way of illustration and not limitation of the invention. Various modifications thereof will occur to those skilled in the art, and such modifications are within the scope of the claims which define the present invention.

As previously explained, an endocardial lead including an electrical conductor encased in an encasing material and having improved fixation characteristics is disclosed. The endocardial lead has four fin-shaped protuberances equally spaced around the circumference of the encasing material near an electrode tip. The trailing edge of each fin defines a structure for deflecting chordae tendonae found in the heart ventricle should it be necessary to relocate the electrode tip after an initial placement. The structure for deflecting the chordae tendonae begins at a point at least ten percent of the distance between the encasing material and the top of the fin and angles backward until it intersects the encasing material. In one embodiment, the deflecting structure defines an arc having a radius equal to the distance between the beginning point of the structure and the encasing material. The fins are formed of a soft pliant material which could be the same as that utilized for the encasing material, and could be silicone rubber or polyurethane. Both of these materials exhibit sufficient rigidity to maintain the fins in an upright position when unrestrained while still allowing the fins to be deformed during placement into or removal from the heart trabeculae. The uniqueness of the deflecting structure allows chordae tendonae present within the heart ventricle to be deflected rather than snagged by the fins during electrode removal or reimplantation operations.

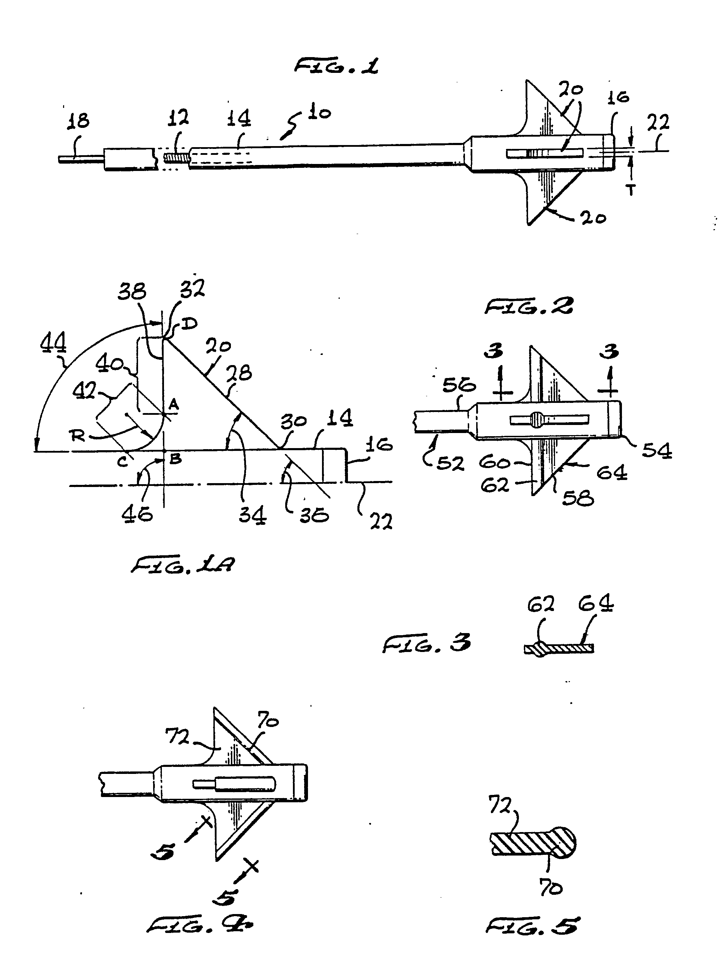

Referring now to Figure 1, an endocardial lead 10 is shown having a centrally-located, spirally-wound electrical conductor 12 encased in an encasing material 14, its distal end terminating in an exposed electrically conductive tip 16. The proximal end 18 of the conductor is adapted to be removably received by an implanted medical device such as a heart pacemaker (not shown). Four fins 20 are equally spaced around the circumference of the encasing material 14 near the electrode tip 16. These fins 20 are chosen to have a unique configuration as will be explained below which provides for firm fixation of the electrode tip 16 within the heart trabeculae while at the same time allowing less traumatic removal of the electrode tip 16 from the heart trabeculae in the event of electrode removal, replacement or reattachment operations. Although an electrode lead of a unipolar configuration is shown in Figure 1, the invention would be equally applicable to a bipolar lead. Further, although an exposed electrically conductive tip 16 is chosen for illustrative purposes, any other body-contact device, such as a temperature sensor, ph sensor, or the like could be utilized. Although the fins 20 are shown in Figure 1 near the electrically conductive tip 16, it should be appreciated that the fins 20 could be located in any position relative to the tip 16 so long as the interaction between the fins 20 and the heart trabeculae result in influencing the location of the tip 16 with respect to the heart wall. The longitudinal axis of the conductor 12 is shown as a dotted line 22.

Referring now to Figure 1A, the fin 20 can be seen enlarged to illustrate its various parts and their relationship with respect to each other. The fin 20 defines an upper edge 28 which begins at a fin distal end 30 and extends upwardly and acutely away from the encasing material 14 to a point defined as a fin proximal end 32. The fin upper edge in the embodiment shown defines an angle 34 with respect to the encasing material 14 equal to forty-five degrees. Further, the fin upper edge 28 substands an angle with respect to the axis 22 also equal to forty-five degrees as shown at 36. Although forty-five degrees for the angles 34 and 36 have been chosen for illustrative purposes, the invention is in no way limited to an upper edge angle of forty-five degrees, and other angles could be utilized just so long as the angle remains acute with respect to the encasing material 14. From the fin proximal end 32, the fin defines a trailing edge 38 which for ease of explanation is defined as a first trailing edge portion 40 and a second trailing edge portion 42. The first trailing edge portion 40 can be seen as the edge defined by segment D-A; the second trailing edge portion 42 can be seen as the edge defined by segment A-C. The vertical distance between the fin proximal end 32 and the encasing material 14 can be seen to be D-B, with the second trailing edge portion 42 beginning at initiation point A. The invention requires that the distance B-A be at least ten percent of the distance B-D for reasons that will be explained in further detail below. However, in the particular embodiment shown, the distance B-A is twenty-five percent of the distance B-D, and leads have been successfully tested in animals in which the distance B-A is fifty percent of the distance B-D. Other distances such as twenty percent could also be utilized.

The second trailing edge portion 42 beginning at point A extends downward and away from the tip 16 until it intersects the encasing material 14 at point C. In the particular embodiment shown, the second trailing edge portion 42 defines an arc having a radius R as shown. Although an arc having a radius R is shown in the illustrative embodiment, it is only necessary for the trailing edge portion to extend generally downward and away from the electrode tip 16. Thus, the second trailing edge portion could define, for example, a straight line extending between A and C, or a stepped line containing a plurality of horizontal and vertical segments connecting points A and C. The requirement for C is that it be at least further from the conductive tip 16 than piont B, and the requirement for point A is that it be at a point at least equal to or greater than ten percent of the distance between points B and D. In one embodiment of the invention, the radius R is equal to 0.125 cm (0.050 inches), the thickness T of the fin is no greater than 0.100 cm (0.040 inches), and the distance B-D shown in Figure 1A is 0.250 cm (0.100 inches). The radius R of 0.125 cm (0.050 inches) was chosen to be substantially equal to the radius of a trabeculae in a human heart ventricle.

In the particular embodiment shown, the angle of the first trailing edge 40 when subtended to intersect the encasing material at B and the conductor axis 22 is chosen to be ninety degrees as shown at 44 and 46. Although ninety degrees for the angles 44 and 46 were chosen, other angles greater than ninety degress could be chosen.

Although in the embodiment shown, the plane of the fin 20 is parallel to the conductor axis 22, the invention is in no way limited to that configuration. For example, the plane of the fin 20 could intersect the encasing material 40 in a way to form an angle with respect to the conductor axis 22.

In operation, a lead having a fixation structure provided by the invention is veneously inserted into a patient. Pressure of the vein will cause the fins 20 to generally deform sideways, thereby tending to decrease the diameter of a circle encompassing the four fins 20. Similarly, once the tip 16 is inserted into the user's ventricle and begins to engage heart trabeculae, the fins will again deform or flex sideways as the tip 16 is pushed between trabeculae to contact the heart wall. Once the tip 16 is located within the trabeculae, the undeformed configuration of each fin 20 will tend to be restored and the fin trailing edge 38 will abut against the heart trabeculae and tend to prevent tip 16 dislodgement. However, should it be necessary for the physician to either explant the lead or reposition the conductive tip 16 to another location within the user's heart, the physician will effect this removal by applying tension along the length of the lead 10. In prior art leads during this removal, chordae tendonae within the user's heart tend to get caught in the intersection between the trailing edge of the fin and the encasing material 14. However, because of the second trailing edge portion 42 formed by the fin in accordance with the teachings of the present invention, the tendency for chordae tendonae to catch is minimized because of the characteristics of the second trailing edge as previously explained.

In the specific embodiment shown, the radius R is chosen to be close to the radius of a trabeculae within the user's heart so as to provide positive fixation or engagement with the trabeculae. However, the radius R is much larger than that of the chordae tendonae so that when the chordae tendonae come in contact with the trailing edge during tip removal, they will tend to slip upward and force the fin 20 to deform in a lateral direction for easy removal, rather than catch in a corner defined by A, B and C shown in Figure 1A as is typical of prior art fins. Thus, the action of chordae tendonae against the fixation device provided by the present invention, rather than inhibiting removal of the electrode tip, actually facilitates removal of the electrode tip by forcing the fin 20 to move in a lateral direction as tension is applied to the lead 10.

Referring again to Figure 1A, another way of characterizing the invention is to consider the fin portion defined by points A, B and C as a chordae tendonae deflection structure with the edge defined by A-C being a specific portion of the structure configured so as to deflect chordae from entangling with the fin defined by the proximal end 30 and points B and D. As previously explained, the portion of the chordae deflection means defined by the edge A-C could be the arc as shown in Figure 1A, a straight edge connecting points A and C, a stepped edge connecting points A and C or any of numerous other edge configurations which slope downward and backward from point A until intercepting the encasing material 14 at point C. A still further way of characterizing the invention is to consider the fin and chordae deflection structure defined by the fin distal end 30 and points D, A and C as a fin-shaped fixation means having the characteristics previously described.

Referring now to Figure 2, a further embodiment of the invention can be seen. Here, an endocardial lead 52 is shown having an exposed electrically conductive tip 54 and encasing material 56 as in the Figure 1 embodiment. The fin upper edge 58 and fin trailing edge 60 have the characteristics as described in conjunction with Figure 1 and Figure 1A. However, the embodiment of Figure 2 incorporates a supporting ridge 62 which is formed of the same pliant material as the fin 64 and is molded into the fin 64 as shown in Figure 3 to provide increased resistance to flexure of the fin 64. As can be appreciated, the supporting ridge 62 is in the form of raised half cylinder surfaces on which side of the fin 64 which will tend to increase the resistance of the fin in moving in a lateral direction with respect to the longitudinal axis of the central conductor in the lead 52. Although in this specific embodiment, the increased flexure is provided by a thickening of the wedge as shown at 62, it should be appreciated that other means of obtaining increased flexure could be utilized such as imbedding a resilient metallic member within the fin 64, or imbedding a nonconductive material within the fin 64 that has more resistance to flexure than the material forming the fin 64. As can be seen, the specific embodiment shown in Figure 2 shows the supporting ridge 62 being located at the proximal end of the fin 64 and oriented so that the longitudinal axis of the raised portion is perpendicular to the surface of the encasing material 56. However, it should be readily appreciated that the supporting ridge could be oriented in anyway within the fin, the specific location being dependent upon the particular flexure characteristics desired.

A still further embodiment can be seen in reference to Figures 4 and 5 in which a supporting ridge 70 is provided along, and defines, the upper edge of the fin 72. As can be appreciated, this is merely another way of altering the characteristics of the fin by providing an upper edge that is more likely to maintain its undeformed shape subsequent to placement within the heart trabeculae.

## Claims

1. An endocardial lead (10, 52) having an electrical conductor (12) encased in an encasing material (14, 56) which is generally inert to body fluids, the conductor (12) terminating in a body-contact means (16, 54), the lead comprising:
a fixation means generally in the form of at least one fin (20, 64, 72) attached to the encasing material (14, 56), said fin (20, 64, 72) having a distal end (30) near the body-contact means (16, 54) and a proximal end, said fin (20, 64, 72) defining an upper edge (28, 58) and a trailing edge (38, 60), the upper edge (28, 58) beginning at the encasing material (14, 56) and extending acutely away from the encasing material (14, 56) from the fin distal end (30), the trailing edge (38, 60) extending vertically or proximally downward from the proximal end of the upper edge (28, 58) with respect to the conductor axis to define a first linear trailing edge portion (40) and, contiguous to said first trailing edge portion, a second trailing edge portion (42), extending proximally away from the first trailing edge portion (42) and downward until it intersects said encasing material (14, 56), said second trailing edge portion (42) beginning at an initiation point (A) no less than ten percent of the perpendicular distance from the surface of the encasing material (14, 56) to the proximal end of the upper edge (28, 58), when the position of the initiation point (A) is measured from the surface of the encasing material (14).

2. The endocardial lead of Claim 1 wherein said initiation point (A) is located at a distance no less than twenty-five percent of the distance from the surface of said encasing material (14, 56) to the proximal end of said upper edge (28, 58).

3. The endocardial lead of Claim 2 wherein said fin (20, 64, 72) is formed of a pliant material having sufficient rigidity to maintain said fin (20, 64, 72) in an upright position when unrestrained, but sufficiently pliant to prevent penetration of heart tissue, said pliant material being generally inert to body fluids.

4. The endocardial lead of Claim 3 wherein said at least one fin (20, 64, 72) comprises four fins equally spaced around the circumference of said encasing material.

5. The endocardial lead of Claim 3 wherein the thickness of said fin (20, 64, 72) is no greater than 0.100 cm (0.040 inches).

6. The endocardial lead of Claim 3 further comprising additional support means (62, 70) for altering the flexure characteristics of said fin (20, 64, 72).

7. The endocardial lead of Claim 6 wherein said additional support means (70) comprises a thickened portion of said pliant material extending along the upper edge of said fin (72).

8. The endocardial lead of Claim 6 wherein said additional support means (62) comprises a thickened portion of said pliant material extending from said encasing material (56) to the proximal end of said upper edge (58).

9. The endocardial lead of Claim 8 wherein said thickened portion (62) forms a substantially ninety degree angle with respect to the surface of said encasing material (56).

10. The endocardial lead of Claim 3 wherein said upper edge (28, 58) forms a substantially forty-five degree angle with respect to the surface of said encasing material (14, 56).

11. The endocardial lead of Claim 3 wherein said upper edge (28) forms a substantially forty-five degree angle with respect to the longitudinal axis (22) of said electrical conductor (12).

12. The endocardial lead of Claim 3 wherein said body-contact means comprises an exposed, electrically conductive tip (16, 54).

13. The endocardial lead of Claim 3 wherein said second trailing edge portion (42) defines a concave arc.

14. The endocardial lead of Claim 13 wherein said concave arc has a radius of substantially 0.125 cm (0.050 inches).

15. The endocardial lead of Claim 3 wherein said first trailing edge portion (40) defines a ninety degree angle with respect to said encasing material (14).

16. The endocardial lead of Claim 3 wherein said first trailing edge portion (28) defines a ninety degree angle with respect to the longitudinal axis (22) of said electrical conductor.

**Patentansprüche**

1. Endokardiale Leitung (10, 22), die einen elektrischen Leiter (12) aufweist, der von einem, allgemein gegenüber Körperflüssigkeiten inerten Hüllmaterial (14, 56) umschlossen ist und in einem den Körper kontaktierenden Mittel (16, 54) endet; dabei weist die Leitung Befestigungsmittel in Form von mindestens einer Flosse (20, 64, 72) auf, die an dem Hüllmaterial (14, 56) befestigt ist und ein distales Ende (30) nahe dem den Körper kontaktierenden Mittel (16, 54) sowie ein proximales Ende aufweist; die Flosse (20, 64, 72) weist eine Oberkante (28, 58) und eine Hinterkante (30, 60) auf; die Oberkante (28, 58) beginnt an dem Hüllmaterial (14, 56) und setzt sich von dem distalen Ende (30) der Flosse aus von dem Hüllmaterial (14, 56) spitz weglaufend fort; die Hinterkante (38, 60) erstreckt sich in bezug auf die Achse des Leiters vertikal oder in proximaler Richtung von dem proximalen Ende der Oberkante (28, 58) aus nach unten, um einen ersten Hinterkantenabschnitt (40) und daran anschliessend einen zweiten Hinterkantenabschnitt (42) zu definieren, der sich in proximaler Richtung von dem ersten Kantenabschnitt (40) weg nach unten erstreckt, bis er das Hüllmaterial (14, 56) schneidet, wobei der zweite Hinterkantenabschnitt (42) an einem Anfangspunkt (A) von nicht weniger als 10% des lotrechten Abstandes von der Oberfläche des Hüllmaterials (14, 56) bis zum proximalen Ende der Oberkante (28, 58) beginnt, wenn die Position des Anfangspunktes (A) von der Oberfläche des Hüllmaterials (14, 56) aus gemessen wird.

2. Endokardiale Leitung nach Anspruch 1, dadurch gekennzeichnet, dass der Anfangspunkt (A) in einem Abstand von nicht weniger als 25% des Abstandes von der Oberfläche des Hüllmaterials (14, 56) bis zu dem proximalen Ende der Oberkante (28, 58) gelegen ist.

3. Endokardiale Leitung nach Anspruch 2, dadurch gekennzeichnet, dass die Flosee (20, 64, 72) aus einem elastischen Material ausgebildet ist, das eine ausreichende Steifigkeit aufweist, um die Flosse (20, 64, 72), wenn sie ungehindert ist, in einer aufrechten Stellung zu halten, aber auch ausreichend elastisch ist, um ein Eindringen in das Herzgewebe zu verhindern, wobei das elastische Material allgemein inert gegenüber Körperflüssigkeiten ist.

4. Endokardiale Leitung nach Anspruch 3, dadurch gekennzeichnet, dass die mindestens eine Flosse (20, 64, 72) vier Flossen umfasst, die äquidistant entlang des Umfanges des Hüllmaterials angeordnet sind.

5. Endokardiale Leitung nach Anspruch 3, dadurch gekennzeichnet, dass die Dicke der Flosse (20, 64, 72) nicht grösser als 0,100 cm (0,040 inches) ist.

6. Endokardiale Leitung nach Anspruch 3, dadurch gekennzeichnet, dass sie weiterhin zusätzliche Stützmittel zur Änderung der Biegecharakteristik der Flosse (20, 64, 72) umfasst.

7. Endokardiale Leitung nach Anspruch 6, dadurch gekennzeichnet, dass die zusätzlichen Stützmittel (70) ein verdicktes, sich entlang der Oberkante der Flosse (72) erstreckendes Teil des elastischen Materials umfassen.

8. Endokardiale Leitung nach Anspruch 6, dadurch gekennzeichnet, dass die zusätzlichen Stützmittel (62) ein verdicktes, sich von dem Hüllmaterial (56) bis zu dem proximalen Ende der Oberkante (28) ersteckendes Teil des elastischen Materials umfassen.

9. Endokardiale Leitung nach Anspruch 8, dadurch gekennzeichnet, dass das verdickte Teil (62) einen Winkel von im wesentlichen 90° zu der Oberfläche des Hüllmaterials (56) bildet.

10. Endokardiale Leitung nach Anspruch 3, dadurch gekennzeichnet, dass die Oberkante (28, 58) einen Winkel von im wesentlichen 45° zu der Oberfläche des Hüllmaterials (14, 56) bildet.

11. Endokardiale Leitung nach Anspruch 3, dadurch gekennzeichnet, dass die Oberkante (28) einen Winkel von im wesentlichen 45° zu der Längsachse (22) des elektrischen Leiters (12) bildet.

12. Endokardiale Leitung nach Anspruch 3, dadurch gekennzeichnet, dass das den Körper kontakttierende Mittel eine freiliegende, elektrisch leitende Spitze (16, 54) umfasst.

13. Endokardiale Leitung nach Anspruch 3, dadurch gekennzeichnet, dass der zweite Hinterkantenabschnitt (42) einen konkaven Bogen bildet.

14. Endokardiale Leitung nach Anspruch 13, dadurch gekennzeichnet, dass der konkave Bogen einen Radius von im wesentlichen 0,125 cm (0,050 inches) aufweist.

15. Endokardiale Leitung nach Anspruch 3, dadurch gekennzeichnet, dass der erste Hinterkantenabschnitt (40) einen Winkel von 90° zu dem Hüllmaterial (14) bildet.

16. Endokardiale Leitung nach Anspruch 3, dadurch gekennzeichnet, dass der erste Hinterkantenabschnitt (28) einen Winkel von 90° zu der Längsachse (22) des elektrischen Leiters bildet.

## Revendications

1. Câble endocardiaque (10, 52) comportant un conducteur électrique (12) compris dans une gaine faite avec un matériau (14, 56) qui est généralement inerte vis-à-vis à des fluides du corps, le conducteur (12) se terminant par des moyens (16, 54) assurant le contact avec le corps, le câble comportant:

des moyens de fixation ayant généralement la forme d'au moins d'une ailette (20, 64, 72) attachée au matériau de la gaine (14, 56), ladite ailette (20, 64, 72) ayant une extrémité distale (30) près des moyens de contact avec le corps (16, 54) et une extrémité proximale, ladite ailette (20, 64, 72) définissant un bord supérieur (28, 58) et un bord arrière (38, 60), le bord supérieur (28, 58) commençant au niveau du matériau constitutif de la gaine (14, 56) et s'éloignant finement du matériau constitutif de la gaine (14, 56) à partir de l'extrémité distale (30) de l'ailette, le bord arrière (38, 60) s'étendant verticalement ou à peu près vers le bas à partir de l'extrémité proximale du bord supérieur (28, 58) par rapport à l'axe du conducteur pour définir une première partie linéaire (40) du bord arrière et, de façon contiguë à ladite première partie linéaire du bord arrière, une seconde partie (42) de bord arrière s'étendant finement en s'éloignant de la première partie (42) du bord arrière et vers le bas jusqu'à intersection avec ledit matériau constitutif de la gaine (14, 56), ladite seconde partie (42) du bord arrière commençant en un point initial ou d'amorçage (A) qui n'est pas inférieur à dix pour-cent de la distance perpendiculaire entre la surface du matériau constitutif de la gaine (14, 56) et l'extrémité proximale du bord supérieur (28, 56) lorsque la position du point d'amorçage (A) est mesurée à partir du matériau constitutif de la gaine (14).

2. Câble endocardiaque selon la revendication 1, dans lequel ledit point d'amorçage (A) est situé à une distance qui n'est pas inférieure à vingt cinq pour-cent de la distance entre la surface dudit matériau constitutif de la gaine (14, 56) et l'extrémité proximale dudit bord supérieur (28, 58).

3. Câble endocardiaque selon la revendication 2, dans lequel ladite ailette (20, 64, 72) est faite avec un matériau flexible présentant une rigidité suffisante pour maintenir ladite ailette (20, 64, 72) dans une position redressée, en l'absence d'une contrainte, mais assez flexible pour empêcher la pénétration du tissu cardiaque, ledit matériau flexible étant généralement inerte par rapoprt aux liquides corporels.

4. Câble endocardiaque selon la revendication 3, dans lequel ladite au moins une ailette (20, 64, 72) comprend quatre ailettes réparties uniformément sur la circonférence dudit matériau constitutif de la gaine.

5. Câble endocardiaque selon la revendication 3, dans lequel l'épaisseur de ladite ailette (20, 64, 72) n'est pas supérieur à 0,100 cm (0,040 ponces).

6. Câble endocardiaque selon la revendication 3, comportant, en outre, des moyens de support supplémentaires (62, 70) pour modifier les caractéristique de flexibilité de ladite ailette (20, 64, 72).

7. Câble endocardiaque selon la revendication 6, dans lequel lesdits moyens de support supplémentaires (70) comprennent une partie épaissie dudit matériau flexible, s'étendant le long du bord supérieur de ladite ailette (72).

8. Câble endocardiaque selon la revendication 6, dans lequel lesdits moyens de support supplémentaires (62) comprennent une partie épaissie dudit matériau flexible, s'étendant du matériau constitutif de la gaine (56) à l'extrémité proximale dudit bord supérieur (58).

9. Câble endocardiaque selon la revendication 8, dans lequel ladite partie épaissie (62) fait sensiblement un angle de quatre vingt dix degrés avec la surface dudit matériau constitutif de la gaine (56).

10. Câble endocardiaque selon la revendication 3, dans lequel ledit bord supérieur (28, 58) forme sensiblement un angle de quarante cinq degrés avec la surface dudit matériau constitutif de la gaine (14, 56).

11. Câble endocardiaque selon la revendication 3, dans lequel ledit bord supérieur (28) forme sensiblement un angle de quarante cinq degrés avec l'axe longitudinal (22) dudit conducteur électrique (12).

12. Câble endocardiaque selon la revendication 3, dans lequel lesdits moyens de contact avec le corps comprennent un embout dénudé électriquement conducteur (16, 54).

13. Câble endocardiaque selon la revendication 3, dans lequel ladite seconde partie (42) du bord arrière détermine un arc concave.

14. Câble endocardiaque selon la revendication 13, dans lequel ledit arc concave possède un rayon sensiblement égal à 0,125 cm (0,050 pouces).

15. Câble endocardiaque selon la revendication 3, dans lequel ladite première partie (40) du bord arrière fait un angle de quatre vingt dix degrés avec ledit matériau constitutif de la gaine (14).

16. Câble endocardiaque selon la revendication 3, dans lequel ladite première partie (28) du bord arrière fait un angle du quatre vingt dix degrés avec l'axe longitudinal (22) dudit conducteur électrique.

FIG.1

FIG.1A

FIG.2

FIG.3

FIG.4

FIG.5